# EUROPEAN PATENT SPECIFICATION

(11) **EP 2 002 842 B1**
(45) Date of publication and mention of the grant of the patent: **11.04.2012**
(21) Application number: 07738057.4
(22) Date of filing: 08.03.2007
(51) Int. Cl.: A23L 1/00, A23K 1/00, A23L 1/30, A61K 35/74

(54) **METHOD FOR PRODUCTION OF THE INTERLEUKIN PRODUCTION REGULATOR**
VERFAHREN ZUR HERSTELLUNG DES INTERLEUKIN-PRODUKTIONSREGLERS
MÉTHODE DE PRODUCTION DE RÉGULATEUR DE PRODUCTION D'INTERLEUKINE

(30) Priority: 31.03.2006 JP 2006099905
(43) Date of publication of application: 17.12.2008
(73) Proprietor: MORINAGA MILK INDUSTRY CO., LTD., Minato-ku, Tokyo 108-8384 (JP)
(72) Inventor: IWABUCHI, Noriyuki, Zama-shi, Kanagawa 228-8583 (JP); SHIMIZU, Kanetada, Zama-shi, Kanagawa 228-8583 (JP); TAKAHASHI, Noritoshi, Zama-shi, Kanagawa 228-8583 (JP)
(74) Representative: Adam, Holger
(86) International application number: PCT/JP2007/054567
(87) International publication number: WO 2007/122885

(56) References cited:
- JP-A- 04 193 832
- JP-A- 05 252 900
- JP-A- 06 056 678
- JP-A- 07 330 806
- JP-A- 08 092 112
- JP-A- 61 257 930
- JP-A- 2000 095 698
- JP-A- 2002 534 113
- JP-A- 2004 262 773
- JP-A- 2005 508 617
- N. IWABUCHI ET AL: "In vitro Th1 cytokine-independent Th2 suppressive effects of bifidobacteria" MICROBIOLOGY AND IMMUNOLOGY, vol. 51, no. 7, 20 July 2007 (2007-07-20), pages 649-660, XP002479195 CENTER FOR ACADEMIC PUBLICATIONS JAPAN ISSN: 0385-5600
- AMROUCHE T. ET AL.: 'Effects of bifidobacterial cytoplasm, cell wall and exopolysaccharide on mouse lymphocyte proliferation and cytokine production' INTERNATIONAL DAIRY JOURNAL vol. 16, no. 1, January 2006, pages 70 - 80, XP005130356

## Description

### TECHNICAL FIELD

The present invention relates to a method for producing an interleukin production regulator having the effect of maintaining or promoting the production of interleukin-10 and the effect of maintaining or inhibiting the production of interleukin-12, which may be used in a pharmaceutical composition and food or drink.

### BACKGROUND ART

The main symptom of autoimmune disease such as rheumatic disease or inflammatory bowel disease is inflammation. It is believed that there are cytokines that cause inflammation and the occurrence of inflammation depends on the balance between cytokines sending signals and cytokine receptors receiving the signals. Further, it is believed that inflammation spontaneously heals when anti-inflammatory cytokines are stronger, but when the action of proinflammatory cytokines is strong, inflammation continues for a long period of time and becomes chronic inflammation. As described above, since it is believed that the balance of production of cytokines (e.g., interleukins) significantly contributes to the development of autoimmune disease, it can be considered that regulation of the production of these cytokines is important to prevent or treat such disease.

IL-10 is a cytokine produced by T cells, B cells, monocytes, and macrophages. IL-10 promotes the proliferation and differentiation of B cells into antibody-secreting cells, and mostly exhibits anti-inflammatory activities. It is believed that this is based on a mechanism where IL-10 upregulates IL-1RA expression by monocytes and inhibits the majority of monocyte inflammatory activities. It has already become apparent that IL-10 inhibits the production of intestinal collagenase and type IV collagenase by interfering with a PGE2-cAMP-dependent pathway, and therefore it is believed that IL-10 can be used as a regulator of connective tissue destruction seen in chronic inflammatory disease. Patent Document 3 discloses Lactococcus strains (especially, Lactococcus lactis subsp. cremoris C60) as microorganisms which efficiently induce the production of IL-10.

IL-12 is a cytokine produced mainly by antigen presenting cells such as macrophages early in the inflammatory cascade, and is a heterodimetric protein of 70 kD composed of 35kD and 40 kD proteins. IL-12 is a strong inducer of IFN-γ production and is also an activator of natural killer cells. It is believed that IL-12 is an important cytokine for generation of cell-mediated, or Th1, immune responses mainly through its ability to activate cells for IFN-γ production. That is, IL-12 generally exhibits proinflammatory activities.

It is known that bifidobacteria exhibit various physiological effects based on their functionality, such as regulation of intestinal functions and reduction of serum cholesterol, when taken into the body in the form of, for example, fermented milk. In recent years, the concept of "probiotics (live microorganisms which beneficially affect host health maintenance)" has been introduced, and therefore such physiological effects of bifidobacteria are receiving broad attention in reflection of consumer health consciousness, and various studies are being conducted. The word "probiotics" was originally defined as "live microbial feed supplements which beneficially affect the host animal by improving its intestinal microbial flora balance", but is now often used in the broad sense described above.

Among various physiological effects of bifidobacteria, effects on autoimmune disease such as insulin-dependent diabetes and chronic rheumatoid arthritis, irritable bowel syndrome, and inflammatory bowel disease such as ulcerative colitis and Crohn's disease have begun to receive attention. In this regard, for example, a therapeutic agent for ulcerative colitis containing the cells of a bifidobacterium as an active ingredient (see Japanese Patent Application Laid-Open No. 7-126177) and Bifidobacterium strains effective for prevention and/or treatment of gastrointestinal inflammatory activity such as inflammatory bowel disease or irritable bowel syndrome (see Japanese Patent Application National Publication No. 2005-508617) have already been known.

Further, it has been already reported that the cells of bifidobacteria stimulate professional antigen presenting cells such as macrophages and dendritic cells to induce IL-12 production (i.e., an IL-12 production-inducing ability) (see International Archives of Allergy and Immunology (Int Arch Allergy Immunol), vol. 135, pp. 205-215, 2004). Further, it has been already reported that bifidobacteria induce IL-10 production (i.e., an IL-10 production-inducing ability) (see Japanese Patent Application National Publication No. 2005-508617). It is believed that the ability of bifidobacteria to induce the production of various interleukins (ILs) varies depending on the kind of strain, and various studies have been conducted until now based on such a way of thinking.
Among further prior art, Japanese Patent Application Laid-Open No. 2005-154387 discloses that cocei of the genus Lactococcus are co-cultured together with the dendritic cells or spleen cells of a mammal to select a strain having high induction properties of the IL-10 production. A selection method for the microorganism or the components comprises culturing microorganisms, for example, the cocei belonging to the genus Lactococcus together with intestinal epithelium cells to select the strain by Caspase-1 activity and IL-18 production inducing ability, food or a food material and animal feed that contain the cocei belonging to the genus Lactococcus as an active ingredient are provided.
Among further prior art, Amrouche et al., International Dairy Journal 16, pp. 70-80 (2006) discloses that bifidobacterial extracts, mainly the cell walls, stimulate the proliferation of lymphocytes and suggest that such extracts could be used in controlling certain immune pathologies.
Among further prior art, JP 2004-262773 discloses a pharmaceutical preparation comprising a bacterial cell ingredient of a bacterium of the genus Bifidobacterium which improves stress resistance and/or dysfunction of the immunological competence induced by the stress. The bifidus bacterial pharmaceutical preparation is useful for improving the dysfunction of the intestinal canal immunological system caused as a result of the stress.
Among further prior art, JP 2000-095698 discloses a remedy for containing a microbial cell component of lactic acid bacteria as an active ingredient. According to this reference, the lactic acid bacteria can be the bacteria belonging to one genus of Lactobacillus, Bifidobacterium, Enterococcus or the like. An extraction method of the microbial cell component is preferably the one of subjecting the microbial cells to lysing treatment by using a lytic enzyme, and further subjecting the treated microbial cells to heating treatment. The cell wall can be destroyed by using a physical method such as an ultrasonic treatment and a French press.
Among further prior art, JP H07-330806 discloses a polysaccharide which is isolated from the cell wall of Bifidobacterium, and which shows specific physical and chemical properties, and induces the increase of granulocytes in white blood cells.
Among further prior art, JP H06-056678 discloses an immunopotentiator containing the cell wall fraction and/or the cytoplasmic fraction of a microorganism belonging to the genus Bifidobacterium as active ingredients. The microorganism is preferably Bifidobacterium adrecientis. The fraction is obtained by centrifuging the cells of anaerobically cultured Bifidobacterium adrecientis at 600G at 5°C for 10 minutes, suspending the cells again in distilled water, centrifuging under the same conditions, washing them, repeating the operations 3 times, breaking the cell bodies with ultrasonic waves for 15 minutes, removing unbroken cells by centrifugation at 800 to 1,0000 at 5°C for 10 minutes and effecting ultracentrifugation with 70,000 G at 5°C for 10 minutes to precipitate the cell wall fraction and collect the cytoplasmic fraction as a supernatant.
Among further prior art, JP H05-252900 describes a mehtod for the production of an immunopotentiate composition, according to which at least one kind of lactic acid bacteria selected from those belonging to Lactobacillus, Bifidobacterium, Pediococcus, Streptococcus, and Leuconostoc is cultured, and the lactic bacteria microbes in the resulting cultured product and/or culture solution are ground. Then, the cytoplasmic fraction of the microbes and/or a product containing the fraction in the resulting supernatant are diluted or concentrated, fractionated, purified and/or dried, thus obtaining a immunopotentiate composition useful for the prevention and treatment of various diseases resulting from various kinds of antigenic substances or infectious microorganisms.

### DISCLOSURE OF THE INVENTION

As described above in the Background Art, it is important to regulate the production of cytokines, especially interleukins, for prevention, treatment, and prevention of recurrence of intestinal disease and autoimmune disease accompanied by inflammation as a main symptom. Among various cytokines, IL-10 as an anti-inflammatory cytokine and IL-12 as a proinflammatory cytokine can be considered important.

It is therefore an object of the present invention to provide a method for producing an interleukin production regulator which can be used for prevention, treatment, and prevention of recurrence of intestinal disease and autoimmune disease accompanied by inflammation as a main symptom and which has both the effect of maintaining or promoting the production of interleukin-10 and the effect of maintaining or inhibiting the production of interleukin-12.

Although not in accordance with the present invention, a method for producing an interleukin production regulator according to the present invention could be used for prevention, treatment, and prevention of recurrence of intestinal disease and autoimmune disease accompanied by inflammation as a main symptom, and could be highly safe and could be administered for a long period of time without fear.

In order to achieve the above objects, the present inventors have searched a raw material which can be used for prevention, treatment, and prevention of recurrence of intestinal disease and autoimmune disease accompanied by inflammation as a main symptom, which is highly safe when used as a pharmaceutical preparation or food or drink, and can be consumed for a long period of time without fear. As a result, they have found that a cell disruption product obtained by carrying out the step of disrupting a microorganism belonging to the genus Bifidobacterium has an interleukin production-regulating ability, having simultaneously the effect of maintaining or promoting the production of interleukin-10 and the effect of maintaining or inhibiting the production of interleukin-12, and this finding has led to the completion of the present invention.

Amazingly, the present inventors have also found that the cell disruption products of any test microorganisms (as starting materials) belonging to the genus Bifidobacterium obtained by carrying out the step of disrupting said microorganism
have such useful interleukin production-regulating ability. That is, such a useful interleukin production-regulating ability can be obtained from a cell disruption product of any microorganism belonging to the genus Bifidobacterium obtained by sufficiently disrupting the cells thereof.

Such amazing findings disprove common knowledge among those skilled in the art which is a premise of the prior art documents such as the Non-Patent Document 1, that is, the common knowledge that the ability of microorganisms belonging to the genus Bifidobacterium to induce the production of various interleukins varies depending on the kind of strain, and therefore in order to use a desired interleukin production-inducing ability, it is necessary to conduct research and development to search a desired strain by screening various strains, and the use of (the cells of a strain having) a desired interleukin production-inducing ability can be realized only after carrying out such screening.

The present invention is directed to a method for producing an interleukin production regulator for maintaining or promoting the production of interleuin-10 and maintaining or inhibiting the production of interleukin-12, the method comprising the step of disrupting a microorganism belonging to the genus Bifidobacterium in suspension carried out by ultrasonic disruption which is accomplished with the energy of 7,800 Joules to 31,500 Joules per milliliter.

In the production method according to the present invention, the interleukin production regulator can achieve a ratio of the production quantity of interleukin-10 acceleratively produced by its effect of maintaining or promoting the production of interleukin-10 to the production quantity of interleukin-12 suppressively produced by its effect of maintaining or inhibiting the production of interleukin-12 (i.e., a ratio of interlukin-10/interleukin-12) of 10 or more, more preferably 20 or more.

Further, the step of disrupting a microorganism is carried out by ultrasonic disruption.

Further, the microorganism belonging to the genus Bifidobacterium is preferably one or more microorganism selected from the group consisting of microorganisms belonging to the species Bifidobacterium longum, microorganisms belonging to the species Bifidobacterium angulatum, microorganisms belonging to the species Bifidobacterium pseudocatenulatum, and microorganisms belonging to the species Bifidobacterium catenulatum.

The present invention may be used for producing an interleukin production regulator obtained by the production method described above, the interleukin production regulator having the effect of maintaining or promoting the production of interleukin-10 and the effect of maintaining or inhibiting the production of interleukin-12.

The interleukin production regulator according to the present invention can achieve a ratio of the production quantity of interleukin-10 acceleratively produced by its effect of maintaining or promoting the production of interleukin-10 to the production quantity of interleukin-12 suppressively produced by its effect of maintaining or inhibiting the production of interleukin-12 (i.e., a ratio of interlukin-10/interleukin-12) of 10 or more, more preferably 20 or more.

Although not in accordance with the present invention, it could be possible to produce an interleukin production regulator which can be used for prevention, treatment, and prevention of recurrence of intestinal disease and autoimmune disease accompanied by inflammation as a main symptom, which has both the effect of maintaining or promoting the production of interleukin-10 and the effect of maintaining or inhibiting the production of interleukin-12. Further, it could also be possible to provide such an interleukin production regulator, a pharmaceutical composition containing the interleukin production regulator as an active ingredient, and a food or drink containing the interleukin production regulator.

Although not in accordance with the present invention, the interleukin production regulator could be useful for prevention, treatment, and prevention of recurrence of intestinal disease and autoimmune disease accompanied by inflammation as a main symptom, that is, autoimmune disease such as insulin-dependent diabetes and chronic rheumatoid arthritis, irritable bowel syndrome, and inflammatory bowel disease such as ulcerative colitis and Crohn's disease.

The interleukin production regulator produced according to the present invention uses as a starting material, the cells of a microorganism belonging to the genus Bifidobacterium, that is, a so-called bifidobacterium. Bifidobacteria have been historically consumed by humans in the form of, for example, yogurt for a very long period of time, and therefore their safety for humans is assured at a very high level. For this reason, the interleukin production regulator, pharmaceutical composition and food or drink are extremely safe, and therefore can be administered or consumed for a long period of time without fear.

### BEST MODE FOR CARRYING OUT THE INVENTION

Hereinbelow, a preferred embodiment of the present invention will be described in detail. However, the present invention is not limited to the following preferred embodiment, and changes can be freely made within the scope of the invention. It is to be noted that "percent" herein refers to "percent by mass" unless otherwise specified.

A strain of the microorganism belonging to the genus Bifidobacterium to be used in the present invention is not particularly limited, and may be one previously deposited as a strain belonging to the genus Bifidobacterium in a public culture collection such as the American Type Culture Collection (ATCC), Japan Collection of Microorganisms (JCM), Northeast Texas Community College (NTCC), or Deutsche Sammlung von Mikroorganismen und Zellkulturen (DSM) or may be one isolated from nature (e.g., from human feces) by such a well-known method as described above. Examples of a strain deposited in public culture collections include FERM BP-7787, ATCC 27535, JCM 7041, and JCM 1194.

As a supply source of the microorganism belonging to the genus Bifidobacterium, a material containing such a microorganism can be used. Examples of such a material include cell suspensions, cell cultures (including cells, culture supernatant, and culture medium components), cell culture solutions obtained by removing solid matter from cell cultures, freeze-dried cell suspensions, freeze-dried cell cultures, and fermented milk using a food or drink fermented by bifidobacteria, such as a drink containing bifidobacteria, acidified milk, and yogurt. The microorganism may be isolated from such a material, or such a material containing the microorganism may be directly used.

The microorganism belonging to the genus Bifidobacterium does not always need to be a single Bifidobacterium strain, and plural strains of the microorganism belonging to the genus Bifidobacterium may be used in combination. Further, the microorganism belonging to the genus Bifidobacterium can be appropriately used in the form of viable cells, wet cells, dried cells, or dead cells.

An active ingredient of the interleukin production regulator obtained according to the present invention is a cell disruption product of a microorganism belonging to the genus Bifidobacterium. The step of disrupting a microorganism belonging to the genus Bifidobacterium included in the method for producing an interleukin production regulator according to the present invention is carried out by physical disruption. Physical disruption is particularly advantageous because, for example, it is not necessary to add any additional substance and therefore safety and security are not impaired. A specific example of a means for disrupting a microorganism includes physical disruption using a French press or a cell disrupter (e.g., Fast Prep FP120 manufactured by Funakoshi Corporation). According to the present invention, ultrasonic disruption by means of e.g. an ultrasonic disrupter (e.g., BRANSON SONIFIER^{®} 450) is used. In this case, physical disruption can be accomplished by ultrasonic disruption treatment at an output of about 35 W (in a case where the amount of a sample suspension is about 4 mL) for 5 minutes or longer (preferably 10 minutes or longer, more preferably 15 minutes or longer and generally 60 minutes or shorter). The physical disruption is accomplished by ultrasonic treatment carried out by giving energy equal to that described above per unit volume. Physical disruption is accomplished by carrying out such treatment that an energy of 7,800 joules or more and 31,500 joules or less is given per milliliter of a sample solution. An ultrasonic frequency to be used in the ultrasonic treatment is generally in the range of 10 to 50 kHz, preferably in the range of 15 to 40 kHz, particularly preferably in the range of 15 to 30 kHz. Examples of a device to be used for the ultrasonic treatment include, in addition to the BRANSON SONIFIER^{®} described above, TITEC VP-5S, VP-15S, and VP-30S and MISONIX ASTRASON^{®} S3000 and XL2020, for example. A larger output of the ultrasonic disrupter tends to be able to sufficiently disrupt a microorganism in a shorter time. The step of disrupting a microorganism can be achieved by such ultrasonic treatment as described above.

The cell disruption product of a microorganism belonging to the genus Bifidobacterium contained as an active ingredient in the aforementioned interleukin production regulator, pharmaceutical composition and food or drink is a natural product and is therefore highly safe when consumed, and is contained in some food products and daily consumed, and has no toxicity, and produces few side effects even when continuously consumed for a long period of time. Therefore, the cell disruption product of a microorganism belonging to the genus Bifidobacterium can be appropriately administered via, for example, the oral route, and can be formed into tablets, capsules, troches, syrups, granules, powders, and the like by known methods. Further, the cell disruption product of a microorganism belonging to the genus Bifidobacterium can be added as an active ingredient to a food product, and can also be processed into a functional food having the effect of preventing and/or treating autoimmune disease or intestinal disease as one aspect of prevention and/or treatment of autoimmune disease or intestinal disease.

Although not in accordance with the present invention, the dose of the cell disruption product of a microorganism belonging to the genus Bifidobacterium as an active ingredient of the interleukin production regulator, pharmaceutical composition and food or drink would vary depending on dosage form, symptom, age, body weight, etc., but could be in the range of 0.1 µg to 0.5 g/kg body weight/day via oral route, preferably in the range of 1 µg to 0.2 g/kg body weight/day, particularly preferably in the range of 10 µg to 50 mg/kg body weight/day in order to effectively obtain its effect of preventing and/or treating autoimmune disease or intestinal disease.

Although not in accordance with the present invention, such a pharmaceutical composition could be produced by, for example, preparing the cell disruption product of a microorganism belonging to the genus Bifidobacterium using any pharmaceutically acceptable additives such as excipients. In the case of preparing such a formulation, the amount of the cell disruption product of a microorganism belonging to the genus Bifidobacterium contained in the formulation is usually in the range of 0.001 to 10% by mass, preferably in the range of 0.01 to 10% by mass. Examples of the additives to be used for preparing the formulation include excipients, binders, disintegrants, lubricants, stabilizers, flavoring agents, diluents, and injectable solvents.

Examples of the excipients include: sugar derivatives such as lactose, sucrose, glucose, mannitol, and sorbitol; starch derivatives such as corn starch, potato starch, α-starch, dextrin, and carboxymethyl starch; cellulose derivatives such as crystalline cellulose, hydroxypropyl cellulose, hydroxypropylmethyl cellulose, carboxymethyl cellulose, and carboxymethylcellulose calcium; gum arabic; dextran; pullulan; silicate derivatives such as light anhydrous silicic acid, synthetic aluminum silicate, and magnesium aluminometasilicate; phosphate derivatives such as calcium phosphate; carbonate derivatives such as calcium carbonate; and sulfate derivatives such as calcium sulfate. Examples of the binders include, in addition to the excipients mentioned above, gelatin; polyvinyl pyrrolidone; and magrogol. Examples of the disintegrants include, in addition to the excipients mentioned above, chemically-modified starch or cellulose derivatives such as crosscarmellose sodium, carboxymethylstarch sodium, and cross-linked polyvinyl pyrrolidone. Examples of the lubricants include talc; stearic acid; metal stearates such as calcium stearate and magnesium stearate; colloidal silica; waxes such as bee gum and spermaceti wax; boric acid; glycol; carboxylic acids such as fumaric acid and adipic acid; sodium calboxylates such as sodium benzoate; sulfates such as sodium sulfate; leucine; lauryl sulfates such as sodium lauryl sulfate and magnesium lauryl sulfate; silicates such as silicic acid anhydride and silicic acid hydrate; and starch derivatives. Examples of the stabilizers include p-hydroxybenzoic esters such as methylparaben and propylparaben; alcohols such as chrolobutanol, benzyl alcohol, and phenylethyl alcohol; benzalkonium chloride; acetic anhydride; and sorbic acid. Examples of the flavoring agents include sweeteners, acidulants, and flavors. Examples of the injectable solvents include water, ethanol, and glycerin.

An example of the indicator of an interleukin production-regulating ability for such a use includes one obtained by simultaneously evaluating the effect of maintaining or promoting the production of interleukin-10 and the effect of maintaining or inhibiting the production of interleukin-12. More specifically, an interleukin production-regulating ability can be expressed as a quantitative ratio of interleukin-10 to interleuin-12 (i.e., a ratio of IL-10/IL-12). The quantitative ratio of interleukin-10 to interleukin-12 (i.e., the ratio of IL-10/IL-l2) can be calculated by, for example, a method described in PNAS, vol. 102. No. 29, pp. 10321 to 10326, 2005.

In a case where the production of interleukin-10 is maintained or promoted and the production of interleukin-12 is maintained or inhibited, the quantitative ratio of interleukin-10/interleukin-12 becomes relatively high (however, a case where the production of IL-10 and the production of IL-12 are both simultaneously maintained is not included). In this regard, it is possible that the production quantity of interleukin-10 or interleukin-12 can be measured not only by an immunologic method such as ELISA but also by a known protein concentration measurement method.

### BRIEF DESCRIPTION OF THE DRAWINGS

Fig. 1 shows graphs of the production quantity of IL-12p70 induced by intact cells or a disrupted-cell product of each strain;
Fig. 2 shows graphs of the production quantity of IL-10 induced by intact cells or a disrupted-cell product of each strain; and
Fig. 3 shows graphs of the production quantity ofIL-12p70 and IL-10 induced by cell products treated with ultrasonic oscillation for different periods of time and a graph of the ratio of IL-10/IL-12.

Hereinbelow, the present invention will be described in more detail with reference to the following examples, but is not limited to these examples.

### (Reference Example 1)

### <Production of tablets containing cell disruption product of Bifidobacterium longum BP-7787>

150 g of powder of cell disruption product of Bifidobacterium longum BP-7787, 100 g of lactulose powder (manufactured by Morinaga Milk Industry Co., Ltd.), 635 g of malt dextrin powder (manufactured by Matsutani Chemical Industry Co., Ltd.), 85 g of skimmed milk powder (manufactured by Morinaga Milk Industry Co., Ltd.), 1 g of stevia sweetener powder (manufactured by San-Ei Gen F.F.I., Inc.), 5 g of yogurt-flavored powder (manufactured by San-Ei Gen F.F.I., Inc.), and 24 g of a glycerin fatty acid ester powdered preparation (manufactured by Riken Vitamin Co., Ltd.) were added and uniformly mixed, and the thus obtained powder mixture was continuously tabletted using a tabletting machine (manufactured by Hata Iron Works Co., Ltd.) at a tabletting speed of 12 tablets/min at a pressure of 9.8 kPa to produce 1800 tablets (about 900 g, 0.5 g per tablet) containing a cell disruption product of Bifidobacterium longum BP-7787 as a symptom-relieving agent and/or a therapeutic agent for inflammatory bowel disease and irritable bowel syndrome.

### (Reference Example 2)

10.8 kg of a whey protein hydrolysate (manufactured by Morinaga Milk Industry Co., Ltd), 36 kg of dextrin (manufactured by Showa Sangyo Co., Ltd.), and small amounts of water-soluble vitamins and minerals were dissolved in 200 kg of water to prepare an aqueous phase in a tank. At the same time, 3 kg of soybean cooking oil (manufactured by Taiyo-yushi Co., Ltd.), 8.5 kg of palm oil (manufactured by Taiyo-yushi Co., Ltd.), 2.5 kg of safflower oil (manufactured by Taiyo-yushi Co., Ltd.), 0.2 kg of lecithin (manufactured by Ajinomoto Co., Inc.), 0.2 kg of fatty acid monoglyceride (manufactured by Kao Corporation), and a small amount of oil-soluble vitamins were mixed and dissolved to prepare an oil phase. The oil phase was added to the aqueous phase contained in the tank, and then they were mixed by stirring. Then, the mixture was heated to 70°C and then further homogenized by a homogenizer at a pressure of 14.7 MPa. Then, the homogenized mixture was sterilized at 90°C for 10 minutes, concentrated, and spray-dried to prepare about 59 kg of an intermediate powder product. To 50 kg of the thus obtained intermediate powder product, 6.8 kg of sucrose (manufactured by Hokuren), 167 g of amino acid mixture powder (manufactured by Ajinomoto Co., Inc.), and 60 g of a cell disruption product of Bifidobacterium longum BP-7787 were added, and they were uniformly mixed to produce about 56 kg of an enteral nutrition powder containing a cell disruption product of Bifidobacterium longum BP-7787 and having the effect of preventing and/or relieving the symptoms of insulin-dependent diabetes.

Hereinbelow, the present invention will be described in detail with reference to the following test examples.

### <Bifidobacteria used in test examples of the present invention>

The species, depository, number, identifying name herein of each Bifidobacterium strain used in the test examples of the present invention were shown in Table 1.

**[Table 1]**

| Species | Depository | Number | Identifying name |
|---|---|---|---|
| *Bifidobacterium longum* | FERM | BP-7787 | BP-7787 |
| *Bifidobacterium angulatum* | ATCC | 27535 | ATCC27535 |
| *Bifidobacterium pseudocatenulatum* | JCM | 7041 | JCM7041 |
| *Bifidobacterium catenulatum* | JCM | 1194 | JCM1194 |

### <Test Method of the Present Invention>

### (Preparation of Intact Cells)

Each of the Bifidobacterium strains was cultured for 16 hours using MRS (de Man Rogasa Sharpe) medium (Difco), and then an obtained culture was washed with PBS (phosphate-buffer saline) twice, and was further washed with distilled water twice, and was then suspended in distilled water and freeze-dried. The freeze-dried suspension was suspended in PBS, and was then subjected to heat treatment at 100°C for 30 minutes to prepare intact cells.

### (Preparation of Disrupted-Cell Product)

Each of the Bifidobacterium strains shown in Table 1 was cultured for 16 hours using MRS (de Man Rogasa Sharpe) medium (Difco), and then an obtained culture was washed with PBS (phosphate-buffer saline) twice, and was further washed with distilled water twice, and was then suspended in distilled water to obtain about 4 mL of a sample. The sample was subjected to ultrasonic treatment on ice using an ultrasonic disrupter (BRANSON SONIFIER^{®} 450) for 60 minutes (output control 4, output: equivalent of about 35 W, frequency: 20 kHz, constant). The sample was centrifuged at 800 x g for 30 minutes to remove intact cells. The thus obtained sample was observed with a speculum to confirm that intact cells did not remain therein, and was then freeze-dried. The freeze-dried sample was suspended in PBS, and was then subjected to heat treatment at 100°C for 30 minutes to prepare a disrupted-cell product.

### (Preparation of Cell Product Treated with Ultrasonic Oscillation)

B. pseudocatenulatum JCM7041 was cultured for 16 hours using MRS (de Man Rogasa Sharpe) medium (Difco), and an obtained culture was washed with PBS (phosphate-buffer saline) twice, and was then suspended in PBS to obtain culture suspensions each having a volume of about 4 mL. The culture suspensions were subjected to ultrasonic treatment on ice using an ultrasonic disrupter (BRANSON SONIFIER^{®} 450) for 0, 5, 15, 30, and 60 minutes, respectively (output control 4, output: equivalent of about 35 W, frequency: 20 kHz, constant), and were then subjected to heat treatment at 100°C for 30 minutes.

### (Preparation of Spleen Cells)

Six-week-old male BALB/c mice (available from Charles River Laboratories) were prepared as experimental animals, and were then dissected at the age of 7 to 9 weeks to extract their spleens. Spleen cells were obtained from the dissected spleens, and were then treated with a red blood cell lysis solution (0.144 M ammonium chloride, 17 mM trisaminomethane, pH 7.65) for 3 minutes to prepare spleen cells from which red blood cells had been removed.

### (Experimental Conditions)

The spleen cells prepared by the method described above with reference to "Preparation of Spleen Cells" were suspended in a medium obtained by adding 10% FCS (Gibco), 100 IU/mL of penicillin, and 0.1 mg/mL of streptomycin to RPMI1640 (SIGMA) to obtain a cell suspension containing 2 × 10⁶ cells per milliliter. Then, 0.5 mL of the cell suspension was mixed with the cell product so that a final concentration thereof became 1 µg/mL or 10 µg/mL, and the thus obtained mixture was cultured in a 48-well microplate (FALCON) at 37°C in the presence of 5% CO₂. After a lapse of 2 days, a culture supernatant was collected to measure cytokines contained in the culture supernatant.

### (Measurement of Cytokines)

The concentration of IL-10 was measured by ELISA using Duo Set (R&D Systems). The concentration of IL-12p70 was measured by concentrating the culture supernatant to 1/10 by ultrafiltration (MultiScreen Ultrcel-10, MILLIPORE) and then performing ELISA using Quantikine (R&D Systems).

### <Test Example 1: Comparison of Ability to Induce Production of IL-12 and IL-10 between Disrupted-Cell Product and Intact Cells>

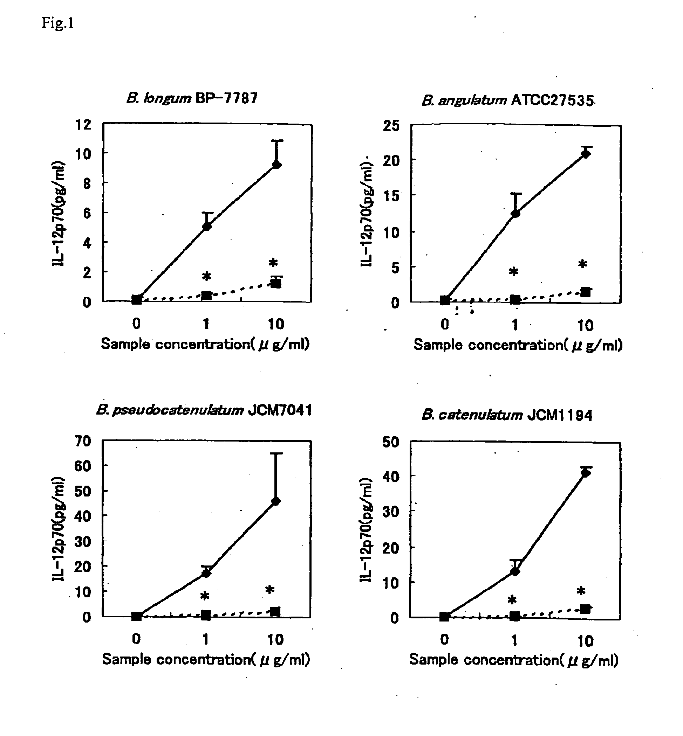

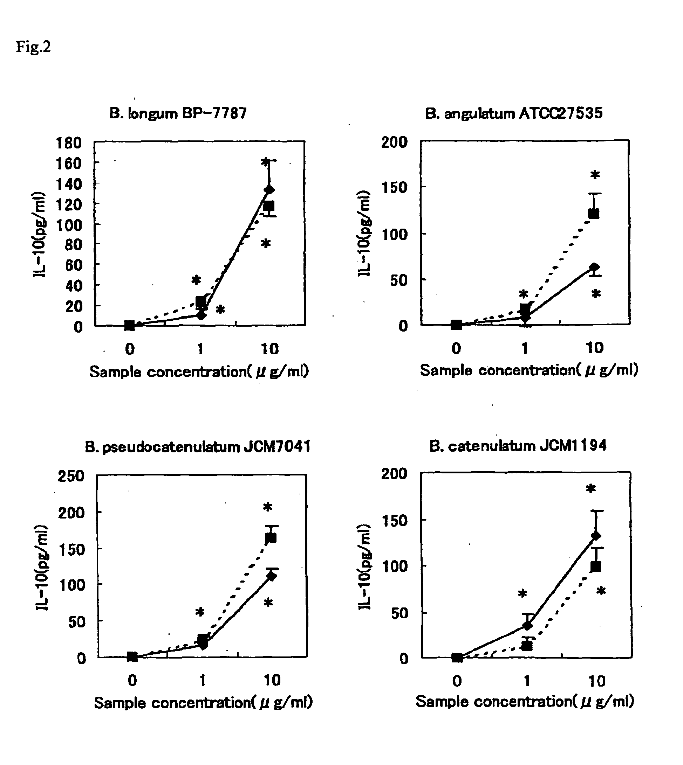

The production quantities of cytokines induced in mouse spleen cells by the intact cells or disrupted-cell product of each of the strains are shown in Fig. 1 (IL-12p70) and Fig. 2 (IL-10).

Fig. 1 shows graphs of the production quantity ofIL-12p70 induced in mouse spleen cells by the addition of the intact cells or disrupted-cell product in the Test Example 1 of the present invention. In Fig. 1, averages of three experiments and standard deviations are shown. Further, the symbol "*" in Fig. 1 indicates that there was a significant difference at a significance level of 5% or less when a case where the intact cells were added (indicated by a solid line) was compared by t-test with a case where the disrupted-cell product was added (indicated by a broken line).

As a result, in all the cases of the strains used in this test, the production quantity ofIL-12p70 induced by the addition of the disrupted-cell product was significantly (t-test at significance level of 5% or less) lower at some sample concentrations as compared to a case where the intact cells were added. This indicates that the ability of the disrupted-cell product to induce the production of IL-12 is lower than that of the intact cells.

Fig. 2 shows graphs of the production quantity of IL-10 induced in mouse spleen cells by the addition of the intact cells or disrupted-cell product in the Test Example 1 of the present invention. In Fig. 2, averages of three experiments and standard deviations are shown. Further, the symbol "*" in Fig. 2 indicates that there was a significant difference at a significance level of 5% or less when a case where the intact cells were added (indicated by a solid line) was compared by t-test with a case where the intact cells were not added (i.e., control) or when a case where the disrupted-cell product was added (indicated by a broken line) was compared by t-test with a case where the disrupted-cell product was not added (i.e., control).

As a result, in all the cases of the strains used in this test, the production quantity of IL-10 was significantly (t-test at significance level of 5% or less) increased by the addition of the intact cells and the disrupted-cell product as compared to a control not containing such a cell product. This indicated that both the intact cells and the disrupted-cell product have the ability to induce the production of IL-10.

The ratio of the production quantity of IL-10 to the production quantity of IL-12p70 (i.e., the ratio of IL-10/IL-12) measured after the addition of the intact cells or the disrupted-cell product of each strain at a concentration of 10 µg/mL is shown in Table 2. In this regard, it is to be noted that in a case where the concentration of IL-12p70 was a detection limit or lower (i.e., 0.25 pg/mL or lower), the production quantity ratio was calculated using the detection limit. In all the cases of the strains, the ratio of IL-10/IL-12 was greatly increased when the disrupted-cell product was added.

**[Table 2]**

| Ratio of IL-10/IL-12 of cytokine induced by intact cells and disrupted-cell product | | | |
|---|---|---|---|
| Species | Strain | Intact cells | Disrupted-cell product |
| *Bifidobacterium longum* | BP-7787 | 14.8 | 107.9 |
| *Bifidobacterium angulatum* | ATCC27535 | 3.0 | 71.9 |
| *Bifidobacterium pseudocatenulatum* | JCM7041 | 2.6 | 71.6 |
| *Bifidobacterium catenulatum* | JCM1194 | 3.2 | 36.0 |

As described above, the ability of the disrupted-cell products of the bifidobacteria to induce the production of IL-12 was lower than that of the intact cells of the bifidobacteria, but the disrupted-cell products and intact cells of the bifidobacteria both had the ability to induce the production of IL-10. This result indicated that the ability of the bifidobacteria to induce the production of IL-12 can be reduced by disrupting their cells while maintaining their ability to induce the production of IL-10. Further, the ratio of IL-10/IL-12 was greatly increased when the disrupted-cell product was added, compared to when the intact cells were added. This indicated that the ratio of IL-10/IL-12 can be significantly improved by disrupting the cells of the bifidobacteria.

### <Test Example 2: Study on Length of Cell Disruption Time>

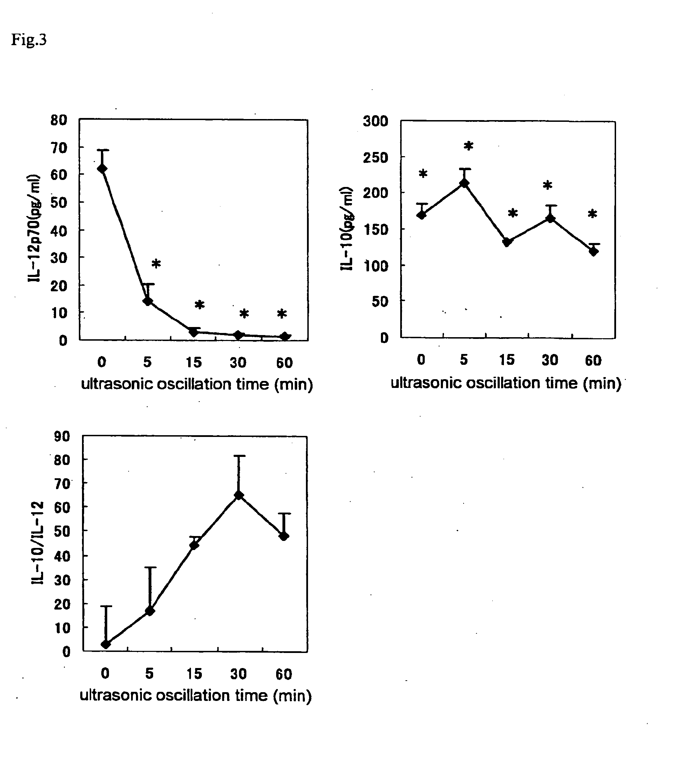

The production quantities of cytokines induced in mouse spleen cells by the addition of the cell products treated with ultrasonic oscillation for different periods of time (sample concentration: 10 µg/mL, B. pseudocatenulatum JCM7041) are shown in Fig. 3.

Fig. 3 shows graphs of the production quantity ofIL-12p70 or IL-10 induced in mouse spleen cells by the addition of the cell products of B. pseudocatenulatum JCM7041 treated with ultrasonic oscillation for different periods of time shown therein and a graph of the ratio of IL-10/IL-12 ("pg/mL"/"pg/mL") in the Test Example 2 of the present invention. In Fig. 3, averages of three experiments and standard deviations are shown. In the graph of the production quantity ofIL-12p70, the symbol "_{*}" indicates that there was a significant difference at a significance level of 0.05 or less when a case where the cell product treated with ultrasonic oscillation was added was compared by t-test with a case where the intact cells (i.e., the cell product treated with ultrasonic oscillation for 0 minute) were added. In the graph of the production quantity of IL-10, the symbol "*" indicates that there was a significant difference at a significance level of 5% or less when a case where the cell product was added was compared by t-test with a control (detection limit or less) not containing the cell product.

As a result, in all the cases where the cell products treated with ultrasonic oscillation for 5 minutes or longer were added, the production quantity of IL-12 was significantly (significance level: 5% or less) reduced, and a longer cell disruption time resulted in a smaller production quantity of IL-12. More specifically, when the cell product treated with ultrasonic oscillation for 15 minutes or longer was added, the production quantity of IL-12 was extremely reduced, and when the cell product treated with ultrasonic oscillation for 30 minutes or longer was added, almost no IL-12 was produced. On the other hand, the production quantity of IL-10 was significantly higher when any one of the cell products treated with ultrasonic oscillation for different periods of time was added as compared to a control not containing the cell product. More specifically, the production quantity of IL-10 was slightly increased by increasing the cell disruption time from 0 to 5 minutes, but after that, tended to be slightly reduced as the cell disruption time was increased. However, all the cell products treated with ultrasonic oscillation basically maintained the ability to induce the production of IL-10 irrespective of the length of cell disruption time.

The above results indicated that the ability of the bifidobacteria to induce the production of IL-12 can be reduced by treating the cells of the bifidobacteria with ultrasonic oscillation using the ultrasonic disrupter (BRANSON SONIFIER^{®} 450) for at least 5 minutes or longer. Further, as can be seen from the graph of the ratio of IL-10/Il-12 in Fig. 3, the cells of the bifidobacteria are preferably treated with ultrasonic oscillation for at least 5 minutes or longer, more preferably 15 minutes or longer and generally 60 minutes or shorter to almost completely block their ability to induce the production of IL-12 and to achieve a high ratio of IL-10/Il-12.

## Claims

1. A method for producing an interleukin production regulator for maintaining or promoting the production of interleukin-10 and maintaining or inhibiting the production of interleukin-12, the method comprising the step of disrupting a microorganism belonging to the genus Bifidobacterium in suspension carried out by ultrasonic disruption which is accomplished with the energy of 7,800 Joules to 31,500 Joules per milliliter.

2. The method according to claim 1, wherein the interleukin production regulator is an interleukin production regulator for increasing the ratio of IL-10 production / IL-12 production.

3. The production method according to claim 1 or 2, wherein the microorganism belonging to the genus Bifidobacterium is one or more microorganism selected from the group consisting of microorganisms belonging to the species Bifidobacterium longum, microorganisms belonging to the species Bifidobacterium angulatum, microorganisms belonging to the species Bifidobacterium pseudocatenulatum, and microorganisms belonging to the species Bifidobacterium catenulatum.

## Patentansprüche

1. Verfahren zur Herstellung eines Interleukinproduktionsregulators zur Aufrechterhaltung oder Begünstigung der Produktion von Interleukin-10 und der Aufrechterhaltung oder Inhibierung der Produktion von Interleukin-12, wobei das Verfahren den Schritt des Zertrennens eines Mikroorganismus, der zu der Gattung Bifidobacterium gehört, in Suspension umfasst, welcher durch Ultraschallzertrennung durchgeführt wird, die mit einer Energie von 7.800 Joule bis 31.500 Joule pro Milliliter erreicht wird.

2. Verfahren gemäß Anspruch 1, wobei der Interleukinproduktionsregulator ein Interleukinproduktionsregulator zur Erhöhung des Verhältnisses IL-10-Produktion/IL-12-Produktion ist.

3. Herstellungsverfahren gemäß Anspruch 1 oder 2, wobei der Mikroorganismus, der zu der Gattung Bifidobacterium gehört, ein Mikroorganismus oder mehrere Mikroorganismen, ausgewählt aus der Gruppe, bestehend aus Mikroorganismen, die zu der Spezies Bifidobacterium longum gehören, Mikroorganismen, die zu der Spezies Bifidobacterium angulatum gehören, Mikroorganismen, die zu der Spezies Bifidobacterium pseudocatenulatum gehören, und Mikroorganismen, die zu der Spezies Bifidobacterium catenulatum gehören, ist.

## Revendications

1. Méthode pour produire un régulateur de production d'interleukine qui a pour effet de maintenir ou de faciliter la production d'interleukine-10 et de maintenir ou d'inhiber la production d'interleukine-12, comprenant l'étape consistant à rompre un microorganisme qui appartient au genre Bifidobacterium, en suspension effectuée utilisant une rupture par ultrasons, qui est accomplie avec une énergie de 7800 joules à 31500 joules par millilitre.

2. Méthode selon la revendication 1, dans laquelle le régulateur de production d'interleukine est un régulateur de production d'interleukine pour augmenter le rapport de production d'IL-10/production d'IL-12.

3. Méthode de production selon la revendication 1 ou 2, dans laquelle le microorganisme qui appartient au genre Bifidobacterium est un ou plusieurs microorganismes choisi(s) parmi le groupe consistant en microorganismes qui appartiennent à l'espèce de Bifidobacterium longum; microorganismes qui appartiennent à l'espèce de Bifidobacterium angulatum; microorganismes qui appartiennent à l'espèce de Bifidobacterium pseudocatenulatum et microorganismes qui appartiennent à l'espèce de Bifidobacterium catenulatum.
